# EUROPÄISCHE PATENTSCHRIFT

(11) **EP 0 682 954 B1**
(45) Veröffentlichungstag und Bekanntmachung des Hinweises auf die Patenterteilung: **28.07.1999**
(21) Anmeldenummer: 95103738.1
(22) Anmeldetag: 15.03.1995
(51) Int. Cl.: A61M 5/32, A61B 17/34, A61B 5/14

(54) **Spinalkanüle mit transparentem Griffteil**
Spinal-cannula with transparent handle
Canule spinale avec poignée transparente

(30) Priorität: 26.03.1994 DE 9405166 U
(43) Veröffentlichungstag der Anmeldung: 22.11.1995
(73) Patentinhaber: Krebs, Peter, Dr., D-78048 Villingen-Schwenningen (DE)
(72) Erfinder: Krebs, Peter, Dr., D-78048 Villingen-Schwenningen (DE)
(74) Vertreter: Neymeyer, Franz, Dipl.-Ing. (FH)

(56) Entgegenhaltungen:
- GB-A- 2 226 496
- US-A- 4 393 879
- US-A- 5 250 035

## Beschreibung

Die Erfindung betrifft eine Spinalkanüle mit einem aus klarsichtig-transparentem Kunststoff bestehenden, das hintere Kanülenende stoffschlüssig umschließenden Griffteil, in dem sich eine von außen sichtbare, sich zum hinteren Kanülenende hin verjüngende Liquor-Kontrollkammer befindet, an die sich rückseitig eine konische Luer-Steckbohrung zur formschlüssigen Aufnahme des Luer-Steckkonus einer Medikamenten-Spritze anschließt, und der außenseitig zwischen zwei Flanschteilen wenigstens zwei sich diametral und symmetrisch zu einer in der Längsachse liegenden Symmetrieebene gegenüberliegende radial vertiefte Grifflächen aufweist, zwischen denen die Liquor-Kontrollkammer angeordnet ist, sowie mit einem Mandrin, der durch die Liquor-Kontrollkammer hindurch in die Kanüle einführbar ist.

Derartige Spinalkanülen werden zur Punktion des Spinalraumes verwendet. Um dabei die korrekte Positionierung der Kanülenspitze im Subarachnoidalraum kontrollieren zu können, ist es wichtig, den Liquor-Rückfluß in der Liquor-Kontrollkammer optisch klar erkennen und kontrollieren zu können. Außerdem soll ein sicheres Halten der Kanüle und gutes Führen gewährleistet sein.

Es sind bereits zahlreiche Spinalkanülen der gattungsgemäßen Art bekannt (Siehe zum Beispiel US-A-5250035), die einen klarsichtigen Griffteil am hinteren Ende der Kanüle aufweisen. Bei diesen bekannten Spinalkanülen ist der Liquor-Kontrollraum des Griffteils bei einigen Ausführungsformen über die gesamte Länge kegelförmig und bei anderen Ausführungsformen lediglich über etwa die halbe axiale Länge kegelförmig und im übrigen zylindrisch ausgebildet. Manche Griffteile sind mit zwei sich diametral gegenüberliegenden muldenartigen, über die gesamte Länge gleichmäßig gewölbten Grifflächen versehen, während andere ebene bzw. stufenweise abgesetzte Grifflächen aufweisen. Außerdem ist eine Spinalkanüle bekannt deren Griffteil eine Vielzahl von Radialrippen aufweist, die in regelmäßigen Axialabständen eine insgesamt kegelförmige Liquor-Kontrollkammer umschließen.

Bei allen bekannten Spinalkanülen der gattungsgemäßen Art treten optische Effekte, wie Spiegelungen, Lichtbrechungen und dgl. Phänomene auf, welche die visuelle Kontrolle des Liquor-Rückflusses innerhalb der Liquor-Kontrollkammer beeinträchtigen.

Außerdem ist die Liquor-Kontrollkammer bei den meisten bekannten Spinalkanülen zu kurz, um über eine ausreichend lange Strecke und Zeitdauer den Liquorfluß kontrollieren zu können.

Der Erfindung liegt die Aufgabe zu Grunde, den Griffteil einer Spinalkanüle so zu gestalten, daß einerseits störende optische Effekte im Bereich der Liquor-Kontrollkammer vermieden werden und dadurch der bei der Duralpunktion auftretende Liquor-Rückfluß optisch gut erkennbar und über eine ausreichend lange Strecke und Zeitdauer kontrollierbar wird und daß andererseits die Kanüle mit dem Griffteil präzise und bequem geführt werden kann.

Gelöst wird diese Aufgabe erfindungsgemäß dadurch, daß die sich wenigstens annähernd über den Axialabstand der beiden Flanschteile erstreckende Liquor-Kontrollkammer eine im wesentlichen rechteckige Querschnittform mit paarweise keilförmig zueinander verlaufenden Wandflächen aufweist, wobei ein Wandflächenpaar symmetrisch zur Symmetrieebene der Grifflächen angeordnet ist und einen Keilwinkel aufweist, der wesentlich kleiner ist als der Keilwinkel des anderen Wandflächenpaars.

Durch die erfindungsgemäß gestaltete Querschnittsform der Liquor-Kontrollkammer und die zumindest annähernd parallele Anordnung der Grifflächen zu dem einen Wandflächenpaar ist sichergestellt, daß der Liquor-Rückfluß innerhalb der Liquor-Kontrollkammer ohne störende Reflexe und Lichtbrechungen sehr gut erkennbar ist, insbesondere wenn der Betrachter etwa senkrecht auf eine der Grifflächen schaut. Außerdem findet in Fließrichtung der Liquor-Flüssigkeit nur eine relativ geringe jedoch gleichmäßig-lineare Querschnittsvergrößerung der Liquor-Kontrollkammer statt, die es dem Betrachter auch ermöglicht, die Gleichmäßigkeit des Liquor-Flusses überwachen bzw. erkennen zu können, was ihm zugleich Aufschluß über die korrekte Position der Kanülenspitze im Subarachnoidalraum gibt.

Die beiden erfindungsgemäß gestalteten Grifflächen erlauben auch eine gute und exakte Führung und ebenso ein leichtes und sicheres Halten des Griffteiles sowohl bei der Punktion als auch beim Einspritzen eines Anästhetikums mittels einer aufgesetzten Spritze.

Weitere vorteilhafte Ausgestaltungen der Erfindung sind Gegenstand der Unteransprüche 2 bis 13.

Anhand der Zeichnung wird im folgenden ein Ausführungsbeispiel der Erfindung näher erläutert. Es zeigt:
- Fig. 1: eine Spinalkanüle mit Mandrin in perspektivischer Ansicht;
- Fig. 2: den Griffteil der Spinalkanüle in Draufsicht;
- Fig. 3: den Griffteil der Spinalkanüle in Seitenansicht;
- Fig. 4: einen Schnitt IV-IV aus Fig. 3;
- Fig. 5: einen Schnitt V-V aus Fig. 4;
- Fig. 6: einen Schnitt VI-VI aus Fig. 5;
- Fig. 7: einen Schnitt VII-VII aus Fig. 4;
- Fig. 8: einen vergrößerten Ausschnitt VIII aus Fig. 5;
- Fig. 9: den linken Teil der Fig. 4 mit eingesetzter Kanüle in etwas vergrößerter Darstellung.

Die in der Zeichnung dargestellte Spinalkanüle 1 weist eine dünne Kanüle (Hohlnadel) 2 mit einer Stechspitze 3 auf, die mit einer Öffnung 4 versehen ist und deren hinterer Endabschnitt 5 in einem einstückigen, aus klarsichtigem Kunststoff bestehenden Griffteil 6 befestigt ist.

Außerdem ist diese Spinalkanüle 1 mit einem Mandrin 7 versehen, der an seinem hinteren Ende einen als kappenartiger Hohlkörper ausgebildeten Handgriff 8 aufweist. Dieser Mandrin ist durch den hohlen Griffteil 6 hindurch in die Kanüle 2 einführbar und zwar soweit, daß durch das vordere Ende des Mandrins 7 die Öffnung 4 an der Stechspitze verschlossen werden kann.

Der Griffteil 6 ist als einstückiges Spritzgußteil hergestellt, er besteht aus Methylmethacrylat-Butadien-Styrol (MBS), das nicht nur glasähnliche optische Eigenschaften, insbesondere eine glasklare Durchsichtigkeit, sondern auch eine hohe, exakt kontrollierbare Fließfähigkeit beim Spritzvorgang hat, so daß bei der Fertigung höchste Präzision gewährleistet werden kann.

An der der Stechspitze 3 der Hohlnadel zugekehrten Stirnseite ist der Griffteil 6 mit einem sog. Luer-Steckkonus 9 versehen, an den sich ein erster zylindrischer Flanschteil 10 größeren Durchmessers anschließt. In einem axialen Abstand **a** von etwa 17 bis 18 mm befindet sich ein zweiter, gleich großer Flanschteil 11, an den sich ein zylindrischer Ansatz 12 anschließt, in dem sich eine Luer-Steckbohrung 13 befindet, die zur formschlüssigen Aufnahme des Luer-Steckkonus einer nicht gezeigten Medikamenten-Spritze dient. Für spezielle Anwendungsfälle ist es aber auch möglich, eine zweite kleinere Kanüle anstelle des Mandrins 7 in die Spinalkanüle 1 einzuführen, die mit dem gleichen Griffteil 6 ausgestattet ist und deren Steckkonus 9 in diese Luer-Steckbohrung 13 hineinpaßt.

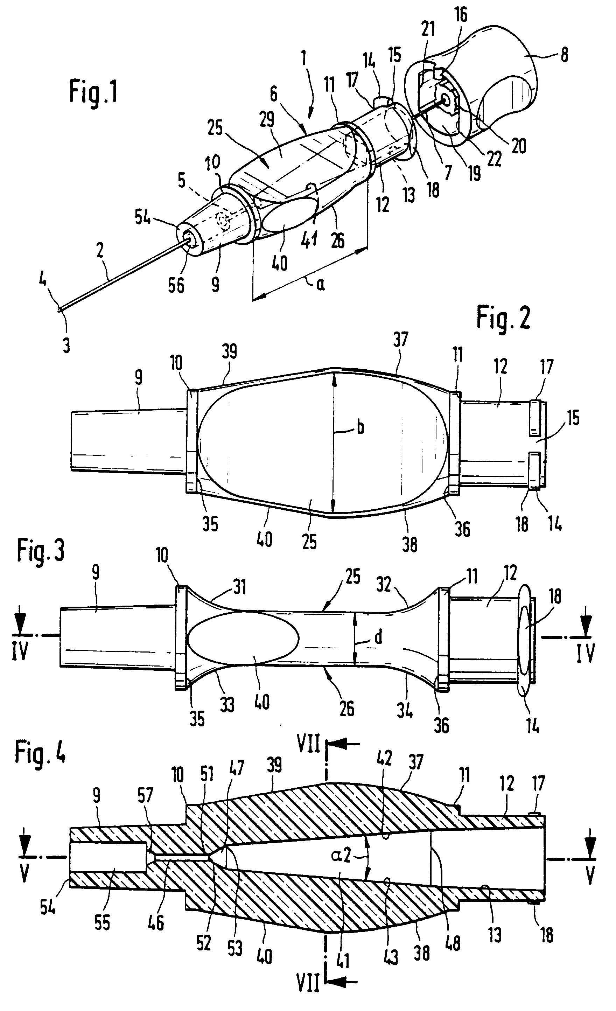

Am hinteren Ende des zylindrischen Endabschnitts 12 ist ein beidseitig mit Abflachungen 17 und 18 versehener Flanschring 14 angeordnet, der auf der in Fig. 1 dargestellten Oberseite eine Ausnehmung 15 aufweist. Dieser Flanschring 14 dient zum winkelmäßigen Ausrichten des Handgriffes 8, der in einer axialen Ausnehmung 19 einen in die Luer-Steckbohrung 13 passenden Zentrierzapfen 20 und eine Führungsrippe 16 aufweist, welche in die Ausnehmung 15 des Flanschringes 14 eingeführt wird, wenn der Handgriff 8 bis zum Flanschteil 11 über den Endabschnitt 12 geschoben wird. Es ist aus Fig. 1 erkennbar, daß auch die Ausnehmung 19 des Handgriffes 8 mit zwei seitlichen Abflachungen 21 bzw. 22 versehen ist, die den Abflachungen 17 und 18 des Flanschringes 14 angepaßt sind.

Das winkelmäßige Ausrichten des Handgriffs 8 und somit auch des Mandrins 7 ist deshalb erforderlich, weil der Mandrin 7 in der Regel an seinem vorderen Ende mit einem der Stechspitze 3 bzw. der Öffnung 4 angepaßten Anschliff versehen ist, der beim Einführen der Stechspitze durch den Epiduralraum in den Spinalraum eine exakt vorgeschriebene Paß-Lage einnehmen muß.

Zwischen den beiden Flanschteilen 10 und 11 befinden sich zwei gegenüber den Flanschteilen 10 und 11 radial vertiefte Grifflächen 25 und 26, die sich diametral gegenüberliegen und symmetrisch zu einer in der Mittelachse 27 des Griffteils 1 liegenden Symmetrieebene 28 angeordnet sind. Diese Grifflächen 25, 26 weisen jeweils einen ebenen, parallel zu dieser Symmetrieebene 28 verlaufenden Mittelabschnitt 29 bzw. 30 auf, an den sich beidseitig jeweils hohlkehlenartige Rundungen 31, 32 bzw. 33, 34 anschließen, die zu den Randkanten 35, 36 der Flanschteile 10 bzw. 11 führen. Auf diese Weise erhalten die Grifflächen 25, 26 eine in Draufsicht insgesamt ellipsenartige Flächenform. Die ebenen, zueinander parallel verlaufenden Mittelabschnitte 29 und 30 erstrecken sich jeweils über eine axiale Länge **a1**, die in der Mitte zwischen den beiden Flanschteilen 10 und 11 liegt. Die Länge **a1** entspricht etwa zwei Drittel von **a**.

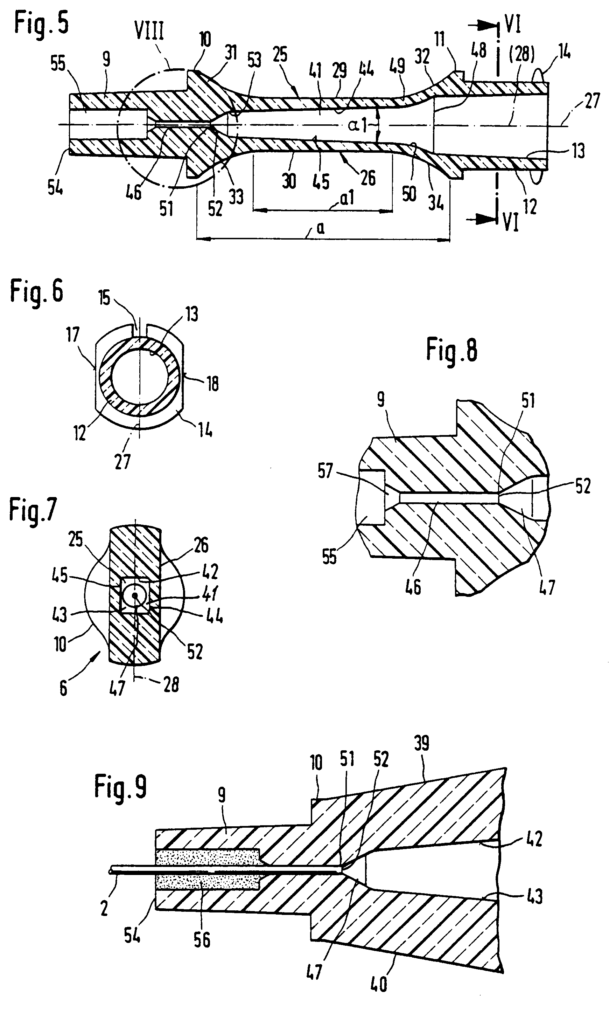

Es ist aus den Fig. 1, 2 und 4 ersichtlich, daß die Grifflächen 25, 26 im Bereich ihrer Mittelabschnitte 29 und 30 jeweils breiter sind als die beiden Flanschteile 10 und 11 und daß der zwischen den beiden Flanschteilen 10, 11 liegende Abschnitt des Griffteils 6 eine Kontur aufweist, die in Draufsicht auf eine der Grifflächen 25, 26 in der hinteren Hälfte jeweils einen Kreisbogen 37 bzw. 38 und in der vorderen Hälfte eine abgeflachte Keilfläche 39 bzw. 40 bildet, wobei auch diese Kontur symmetrisch ist zur Mittelachse 27 (Fig. 5 u. 6).

In Innern des Griffteils 6 befindet sich zwischen den beiden Flanschteilen 10 und 11 eine Liquor-Kontrollkammer 41, die sich zumindst annähernd über den gesamten Abstand **a** erstreckt und eine im wesentlichen rechteckige Querschnittsform mit paarweise keilförmig zueinander verlaufenden, ebenen Wandflächen 42, 43 bzw. 44, 45 aufweist. Dabei ist das Wandflächenpaar 44/45 symmetrisch zu der Symmetrieebene 28 angeordnet, zu der auch die beiden Grifflächen 25 und 26 symmetrisch angeordnet sind. Während die beiden Wandflächen 44 und 45 einen Keilwinkel **α1** von etwa 4° zueinander bilden, weisen die beiden anderen jeweils rechtwinklig dazu verlaufenden Wandflächen 42 und 43 einen mehr als doppelt so großen Keilwinkel **α2** von etwa 10° auf.

Die Raumform der Liquor-Kontrollkammer 41 entspricht somit zumindest annähernd der Raumform einer geraden Pyramide mit rechteckiger Grundfläche und abgeschnittener Spitze.

Es ist aus Fig. 7 ersichtlich, daß die insgesamt breiteren Wandflächen 44 und 45, abgesehen vom Keilwinkel **α1** insgesamt jeweils parallel zu einer der beiden Grifflächen 25 bzw. 26 verlaufen, während die schmäleren Wandflächen 42 und 43 sich rechtwinklig zu diesen Grifflächen erstrecken.

Dadurch werden bei Betrachten der Liquor-Kontrollkammer 41 durch eine der beiden Grifflächen 25, 26 störende optische Phänomene wie Reflexe und Lichtbrechungen vermieden.

Die über ihre gesamte axiale Länge keilförmige Liquor-Kontrollkammer 41 beginnt in der Nähe des vorderen, kanülenseitigen Flanschteiles 10 an einer zentralen Axialbohrung 46, welche den hinteren Endabschnitt 5 der Kanüle 2 passend aufnimmt, mit einem kurzen, kegelförmigen Abschnitt 47, und sie mündet im Bereich des hinteren Flanschteiles 11 unmittelbar in die rückseitige, konische Luer-Steckbohrung 13. Dabei verlaufen die beiden Wandflächen 42, 43 mit dem größeren Keilwinkel **α2** jeweils vom kegelförmigen Abschnitt 47 bis zur gemeinsamen Kante 48, während die beiden anderen Wandflächen 44, 45 mit dem kleineren Keilwinkel **α1** jeweils mit einer kurzen sphärischen Rundung 49 bzw. 50 an diese Steckbohrung 13 anschließen.

Um beim Einführen der Kanüle 2 in die ihren Durchmesser angepaßte Axialbohrung 46 einen axialen Anschlag für deren hinteres Ende zu erhalten, ist diese Axialbohrung 46 mit einer radialen Anschlagschulter 51 versehen, die dadurch gebildet wird, daß der kegelförmige Abschnitt 47 in einer zentralen Bohrung 52 endet, deren Durchmesser kleiner ist als der Durchmesser der Axialbohrung 47 und der höchstens gleich groß ist, vorzugsweise aber etwas kleiner ist als der Innendurchmesser der Kanüle 2. Dadurch ist sichergestellt, daß der von der Rückseite her durch die Liquor-Kontrollkammer 41 hindurch in die Kanüle 2 einzuführende Mandrin 7 sicher in den Rohrhohlraum der Kanüle gelangt, ohne daß dessen Spitze an irgendeiner Kante hängenbleiben kann.

Bei einer bevorzugten Ausführungsform haben die Axialbohrung 46 und die Kanüle 2 einen Durchmesser von 0,42 mm und die Bohrung 52 einen Durchmesser von 0,25 mm, während der Innendurchmesser der Kanüle etwa 0,3 mm beträgt.

Zweckmäßigerweise beträgt die axiale Länge des kegelförmigen Abschnittes 47 etwa 1 mm bis 2 mm, wobei in vorteilhafter Weise zwischen den ebenen Wandflächen 42 und 43 bzw. 44 und 45 und dem kegelförmigen Abschnitt 47 ein abgerundeter Übergang 53 vorgesehen ist. Zwischen der den hinteren Endabschnitt 5 der Kanüle 2 passend aufnehmenden Axialbohrung 47 und der vorderen Stirnseite 54 des Luer-Steckkonus 9 ist eine erweiterte zylindrische, stirnseitig offene Kleberkammer 55 vorgesehen, die zur Befestigung der Kanüle 2 im Griffteil 6 mit einem geeigneten, vorzugsweise Zweikomponentenkleber 56 ausgefüllt wird.

Um das Einführen des hinteren Endabschnitts 5 der Kanüle 2 in die Axialbohrung 46 zu erleichtern, ist das vordere Ende der Axialbohrung 46 mit einer konischen Erweiterung 57 versehen.

Bei einem bevorzugten Ausführungsbeispiel beträgt der Abstand **a** und somit die Gesamtlänge der beiden Grifflächen 25 und 26 etwa 16,5 mm, bei einer Breite **b** von etwa 9,5 mm. Die Dicke **d** im Bereich der Mittelabschnitte 29 und 30 der Grifflächen 25 und 26 beträgt bei diesem bevorzugten Ausführungsbeispiel etwa 3,5 mm.

## Patentansprüche

1. Spinalkanüle (1) mit einem aus klarsichtig-transparentem Kunststoff bestehenden, das hintere Kanülenende (5) stoffschlüssig umschließenden Griffteil (6), in dem sich eine von außen sichtbare, sich zum hinteren Kanülenende hin verjüngende Liquor-Kontrollkammer (41) befindet, an die sich rückseitig eine konische Luer-Steckbohrung (13) zur formschlüssigen Aufnahme des Luer-Steckkonus einer Medikamenten-Spritze anschließt, und der außenseitig zwischen zwei Flanschteilen (10,11) wenigstens zwei sich diametral und symmetrisch zu einer in der Längsachse (27) liegenden Symmetrieebene gegenüberliegende radial vertiefte Grifflächen (25,26) aufweist, zwischen denen die Liquor-Kontrollkammer angeordnet ist, sowie mit einem Mandrin (7), der durch die Liquor-Kontrollkammer hindurch in die Kanüle einführbar ist
**dadurch gekennzeichnet**,
daß die sich wenigstens annähernd über den Axialabstand der beiden Flanschteile (10, 11) erstreckende Liquor-Kontrollkammer (41) eine im wesentlichen rechteckige Querschnittform mit paarweise keilförmig zueinander verlaufenden, ebenen Wandflächen (42, 43, 44, 45) aufweist, wobei ein Wandflächenpaar (44, 45) symmetrisch zur Symmetrieebene (28) der Grifflächen (25, 26) angeordnet ist und einen Keilwinkel (α1) aufweist, der wesentlich kleiner ist als der Keilwinkel (α2) des anderen Wandflächenpaars (42, 43).

2. Spinalkanüle nach Anspruch 1, dadurch gekennzeichnet, daß die Grifflächen jeweils ebene Mittelabschnitte (29, 30) aufweisen, die zueinander parallel verlaufen und an die sich jeweils hohlkehlenartige Rundungen (31, 32, 33, 34) anschließen, die zu den Randkanten (35, 36) der Flanschteile (10, 11) führen.

3. Spinalkanüle nach Anspruch 2, dadurch gekennzeichnet, daß die Grifflächen (25, 26) im Bereich ihrer ebenen Mittelabschnitte (29, 30) breiter sind als die beiden Flanschteile (10, 11), wobei die größte Breite (b) der Grifflächen (25, 26) etwa in ihrer axialen Mitte liegt.

4. Spinalkanüle nach Anspruch 2 oder 3, dadurch gekennzeichnet, daß die Grifflächen (25, 26) eine in Draufsicht insgesamt elliptische Flächenform aufweisen.

5. Spinalkanüle nach Anspruch 1, dadurch gekennzeichnet, daß der zwischen den beiden Flanschteilen (10, 11) liegende Abschnitt des Griffteils (6) eine Kontur aufweist, die sich in Draufsicht auf eine der Grifflächen (25, 26) teils kreisbogenförmig (37, 38), teils keilförmig (39, 40) darstellt.

6. Spinalkanüle nach Anspruch 1, dadurch gekennzeichnet, daß die über ihre gesamte axiale Länge keilförmige Liquor-Kontrollkammer (41) in der Nähe des vorderen, kanülenseitigen Flanschteiles (10) an einer zentralen Axialbohrung, in welche der hintere Endabschnitt (5) der Kanüle (2) eingesetzt ist, mit einem kurzen kegelförmigen Abschnitt (47) von etwa 1 mm bis 2 mm Länge beginnt und im Bereich des hinteren Flanschteiles (11) unmittelbar in die rückseitige, konische Luer-Steckbohrung (13) mündet, wobei die beiden Wandflächen (42, 43) mit dem größeren Keilwinkel (α2) jeweils vom kegelförmigen Abschnitt (47) bis zur gemeinsamen Kante (48) mit der konischen Luer-Steckbohrung (13) gerade verlaufen, während die beiden Wandflächen (44, 45) mit dem kleineren Keilwinkel (α1) jeweils mit einer kurzen sphärischen Rundung (49, 50) an die Luer-Steckbohrung (13) anschließen.

7. Spinalkanüle nach Anspruch 6, dadurch gekennzeichnet, daß die den hinteren Kanülenabschnitt (5) aufnehmende Axialbohrung an einer radialen Anschlagschulter (51) endet.

8. Spinalkanüle nach Anspruch 6 oder 7, dadurch gekennzeichnet, daß der kegelförmige Abschnitt (47) kanülenseitig in einer zentralen Bohrung (52) endet, deren Durchmesser kleiner ist als der Durchmesser der den hinteren Kanülenabschnitt (5) aufnehmenden Axialbohrung (47) und höchstens gleich groß ist wie der Innendurchmesser der Kanüle (2).

9. Spinalkanüle nach einem der Ansprüche 6 bis 8, dadurch gekennzeichnet, daß sich zwischen den geraden Wandflächen (42, 43, 44, 45) und dem kegelförmigen Abschnitt (47) jeweils kurze Rundungsübergänge (53) befinden.

10. Spinalkanüle nach einem der Ansprüche 1 bis 9, dadurch gekennzeichnet, daß der kleinere Keilwinkel (α1) etwa 2° bis 5°, insbesondere 4° und der größere Keilwinkel (α2) etwa 8° bis 12°, insbesondere 10° beträgt.

11. Spinalkanüle nach einem der Ansprüche 1 bis 10, dadurch gekennzeichnet, daß die Grifflächen (25, 26) eine axiale Länge (a) von etwa 16,5 mm und eine maximale Breite (b) von etwa 9,5 mm aufweisen, wobei die Griffdicke (d) im Bereich der Mittelabschnitte (29, 30) der Grifflächen etwa 3,5 mm beträgt.

12. Spinalkanüle nach einem der Ansprüche 1 bis 11, dadurch gekennzeichnet, daß der Griffteil (6) an seinem vorderen, kanülenseitigen Ende mit einem Luer-Steckkonus (9) versehen ist.

13. Spinalkanüle nach einem der Ansprüche 1 bis 12, dadurch gekennzeichnet, daß der Griffteil (6) einstückig aus einem Methylmethacrylat-Polymerisat, insbesondere aus Methylmethacrylat-Butadien-Styrol (MBS) besteht.

## Claims

1. Spinal cannula (1) with a handle part (6) which consists of transparent plastics material, positively surrounds the rear end (5) of the cannula and in which there is located a liquor control chamber (41) which is visible from the exterior, tapers toward the rear end of the cannula, is attached, at its rear, to a conical Luer plug-in socket (13) for the interlocking reception of the Luer plug-in cone of a medication syringe and, externally between two flange parts (10, 11), comprises at least two radially recessed handle faces (25, 26) which are diametrically opposed symmetrically about a plane of symmetry located in the longitudinal axis (27) and between which the liquor control chamber is arranged, and with a stylet (7) which can be introduced through the liquor control chamber into the cannula, characterized in that the liquor control chamber (41) extending at least substantially over the axial distance between the two flange parts (10, 11) has a substantially rectangular cross-sectional shape with plane wall faces (42, 43, 44, 45) converging in pairs in the form, of a wedge, one pair of wall faces (44, 45) being arranged symmetrically about the plane of symmetry (28) of the handle faces (25, 26) and having a wedge angle (α1) which is substantially smaller than the wedge angle (α2) of the other pair of wall faces (42, 43).

2. Spinal cannula according to claim 1, characterized in that the handle faces each comprise plane central portions (29, 30) which extend parallel to one another and are attached to respective groove-like rounded regions (31, 32, 33, 34) which lead to the marginal edges (35, 36) of the flange parts (10, 11).

3. Spinal cannula according to claim 2, characterised in that the handle parts (25, 26) are wider in the region of their plane central portions (29, 30) than the two flange parts (10, 11), the maximum width (b) of the handle faces (25, 26) being located substantially in the axial centre thereof.

4. Spinal cannula according to claim 2 or 3, characterised in that the handle faces (25, 26) have a face form which is elliptical overall in a plan view.

5. Spinal cannula according to claim 1, characterised in that the portion of the handle part (6) located between the two flange parts (10, 11) has a contour which is partially arcuate (37, 38) and partially wedge-shaped (39, 40) in a plan view of one of the handle faces (25, 26).

6. Spinal cannula according to claim 1, characterised in that the liquor control chamber (41) which is wedge-shaped over its entire axial length begins in the vicinity of the front cannula-side flange part (10) at a central axial passage into which the rear end portion (5) of the cannula (2) is inserted with a short conical portion (47) having a length of about 1 mm to 2 mm and opens in the region of the rear flange part (11) directly into the rear conical Luer plug-in socket (13), the two wall faces (42, 43) extending straight with the greater wedge angle (α2) in each case from the conical portion (47) to the common edge (48) with the conical Luer plug-in socket (13), whereas the two wall faces (44, 45) with the smaller wedge angle (α1) are each connected by a short spherical rounded region (49, 50) to the Luer plug-in socket (13).

7. Spinal cannula according to claim 5, characterised in that the axial passage receiving the rear cannula portion (5) ends at a radial stop shoulder (51).

8. Spinal cannula according to claim 6 or 7, characterised in that the conical portion (47) ends on the cannula side in a central passage (52) of which the diameter is smaller than the diameter of the axial passage (47) receiving the rear cannula portion (5) and is at cost equally as great as the internal diameter of the cannula (2).

9. Spinal cannula according to one of claims 6 to 8, characterised in that short rounded transitions (53) are located in each case between the straight wall faces (42, 43, 44, 45) and the conical portion (47).

10. Spinal cannula according to one of claims 1 to 9, characterised in that the smaller wedge angle (α1) is roughly 2° to 5°, in particular 4°, and the greater wedge angle (α2) is about 8° to 12°, in particular 10°.

11. Spinal cannula according to one of claims 1 to 10, characterized in that the handle faces (25, 26) have an axial length (a) of about 16.5 mm and a maximum width (b) of about 9.5 mm, the handle thickness (d) in the region of the central portions (29, 30) of the handle faces being about 3.5 mm.

12. Spinal cannula according to one of claims 1 to 11, characterised in that the handle part (6) is provided with a Luer plug-in cone (9) at its front cannula-side end.

13. Spinal cannula according to one of claims 1 to 12, characterised in that the handle part (6) is formed integrally from a methylmethacrylate polymer, in particular from methylmethacrylate butadiene styrene (MBS).

## Revendications

1. Canule spinale (1) comportant une partie de préhension (6) en plastique transparent qui entoure avec contact de la matière l'extrémité arrière (5) de la canule et dans laquelle se trouve une chambre de contrôle de liqueur (41) qui est visible de l'extérieur, se rétrécit vers l'extrémité arrière de la canule et à laquelle se joint du côté arrière un alésage d'emmanchement Luer conique (13) destiné à recevoir de manière ajustée le cône d'emmanchement Luer d'une seringue à médicaments, cette partie de préhension (6) présentant sur le côté extérieur, entre deux parties brides (10, 11), au moins deux surfaces de préhension approfondies radialement (25, 26) opposées diamétralement et symétriquement par rapport à un plan de symétrie passant par l'axe longitudinal (27) et entre lesquelles est placée la chambre de contrôle de liqueur, ainsi qu'un mandrin (7) qui peut être introduit dans la canule par la chambre de contrôle de liqueur, caractérisée par le fait que la chambre de contrôle de liqueur (41), qui s'étend au moins approximativement sur la distance axiale entre les deux parties brides (10, 11), présente une section sensiblement rectangulaire avec surfaces de paroi planes (42, 43, 44, 45) convergeant deux à deux, deux surfaces de paroi appariées (44, 45) étant placées symétriquement par rapport au plan de symétrie (28) des surfaces de préhension (25, 26) et faisant un angle de coin (α1) sensiblement inférieur à l'angle de coin (α2) des deux autres surfaces de paroi appariées (42, 43).

2. Canule spinale selon la revendication 1, caractérisée par le fait que les surfaces de préhension présentent chacune une partie centrale plane (29, 30), les parties centrales planes s'étendent parallèlement et il s'y joint des arrondis du genre congé (31, 32, 33, 34) qui conduisent aux bords (35, 36) des parties brides (10, 11).

3. Canule spinale selon la revendication 2, caractérisée par le fait que les surfaces de préhension (25, 26), dans la zone de leurs parties centrales planes (29, 30), sont plus larges que les deux parties brides (10, 11), les surfaces de préhension (25, 26) ayant leur plus grande largeur (b) à peu près à leur milieu axial.

4. Canule spinale selon l'une des revendications 2 et 3, caractérisée par le fait que les surfaces de préhension (25, 26), vues de dessus, sont dans l'ensemble elliptiques.

5. Canule spinale selon la revendication 1, caractérisée par le fait que la partie de la partie de préhension (6) située entre les deux parties brides (10, 11) présente un contour qui, sur la vue de dessus d'une des surfaces de préhension (25, 26), est en partie en forme d'arc de cercle (37, 38) et en partie en forme de coin (39, 40).

6. Canule spinale selon la revendication 1, caractérisée par le fait que la chambre de contrôle de liqueur (41), en forme de coin sur toute sa longueur axiale, commence à proximité de la partie bride avant côté canule (10) à un alésage axial central, dans lequel est engagée la partie d'extrémité arrière (5) de la canule (2), par une courte partie conique (47) d'environ 1 mm à 2 mm de long, et débouche dans la zone de la partie bride arrière (11) directement dans l'alésage d'emmanchement Luer conique côté arrière (13), les deux surfaces de paroi (42, 43) qui forment le plus grand angle de coin (α2) s'étendant chacune droit de la partie conique (47) à l'arête commune (48) avec l'alésage d'emmanchement Luer conique (13), tandis que les deux surfaces de paroi (44, 45) qui forment le plus petit angle de coin (α1) se raccordent chacune à l'alésage d'emmanchement Luer (13) par un court arrondi sphérique (49, 50).

7. Canule spinale selon la revendication 6, caractérisée par le fait que l'alésage axial qui reçoit la partie arrière (5) de la canule se termine à un épaulement radial de butée (51).

8. Canule spinale selon l'une des revendications 6 et 7, caractérisée par le fait que la partie conique (47) se termine du côté canule dans un alésage central (52) dont le diamètre est inférieur au diamètre le l'alésage axial (47) qui reçoit la partie arrière (5) de la canule, et au plus égal au diamètre intérieur de la canule (2).

9. Canule spinale selon l'une des revendications 6 à 8, caractérisée par le fait qu'entre les surfaces de paroi droites (42, 43, 44, 45) et la partie conique (47) se trouvent de courts raccords arrondis (53).

10. Canule spinale selon l'une des revendications 1 à 9, caractérisée par le fait que le petit angle de coin (α1) est d'environ 2° à 5°, en particulier de 4°, et le grand angle de coin (α2) est d'environ 8° à 12°, en particulier de 10°.

11. Canule spinale selon l'une des revendications 1 à 10, caractérisée par le fait que les surfaces de préhension (25, 26) ont une longueur axiale (a) d'environ 16,5 mm et une largeur maximale (b) d'environ 9,5 mm, l'épaisseur de préhension (d) dans la zone des parties centrales (29, 30) des surfaces de préhension étant d'environ 3,5 mm.

12. Canule spinale selon l'une des revendications 1 à 11, caractérisée par le fait que la partie de préhension (6) est pourvue d'un cône d'emmanchement Luer (9) à son extrémité avant côté canule.

13. Canule spinale selon l'une des revendications 1 à 12, caractérisée par le fait que la partie de préhension (6) est constituée d'une seule pièce en polymère de méthacrylate de méthyle, en particulier en méthacrylate de méthylebutadiène-styrène (MBS).
